# FASCICULE DE BREVET EUROPEEN

(11) **EP 0 815 829 B1**
(45) Date de publication et mention de la délivrance du brevet: **03.07.2002**
(21) Numéro de dépôt: 97401418.5
(22) Date de dépôt: 19.06.1997
(51) Int. Cl.: A61K 7/06, A61K 7/48

(54) **Utilisation de dérivés amino-alcools à fonction urée dans des compositions cosmétiques ou dermatologiques**
Verwendung von Amino-Alkoholen mit einer Harnstoff-gruppe in kosmetischen und dermatologischen Mitteln
Use of amino-alcohol derivatives containing an urea function in cosmetic and dermatologic compositions

(30) Priorité: 01.07.1996 FR 9608173
(43) Date de publication de la demande: 07.01.1998
(60) Demande divisionnaire: 02290573.1
(73) Titulaire: L'OREAL, 75008 Paris (FR)
(72) Inventeur: Philippe, Michel, 91320 Wissous (FR); Blaise, Christian, 94160 Saint Mande (FR); Tuloup, Rémy, 75014 Paris (FR)
(74) Mandataire: Miszputen, Laurent

(56) Documents cités:
- EP-A- 0 383 024
- DE-A- 2 703 185
- NL-A- 7 414 311
- US-A- 3 149 042
- WILLIAMS ET AL.: "urea analogues in propylene glycol as penetration enhancers in human skin" INTERNATIONAL JOURNAL OF PHARMACEUTICS, vol. 56, no. 1, 1 novembre 1989, NL, pages 43-50, XP000645464

## Description

La présente invention concerne l'utilisation de dérivés amino alcools à fonction urée comme agents de conditionnement dans des produits capillaires.

On connaît dans l'état de la technique un certain nombre de composés amino-alcools à fonction urée. Certains ont été notamment décrits dans le brevet US 3.135.790. et sont utilisés comme agents émulsifiants dans des formulations de peintures ou de laques ou comme intermédiaires de synthèse. D'autres tels que ceux décrits dans le brevet DE 1 125 905, notamment le N-méthyl-N-(2,3,4,5,6-penta-hydroxy-n-hexyl)-N'-n-octyl-urée, sont utilisés comme additifs pour textiles, agents de lavage, agents de réticulation ou comme émulsifiants. On utilise certains dérivés amino-alcools à fonction urée dans le brevet US 2.891.944 pour leurs propriétés insecticides. On a décrit également le 3-(3-octadécyluréido)-1,2-propanediol dans l'article J. Med. Chem. 31(4), 858-863, 1988, pour son activité sur certaines cellules cancéreuses.

La Demanderesse a découvert de manière surprenante que des dérivés amino-alcools à fonction urée particuliers dont on définiera la structure ci-dessous présentaient des propriétés cosmétiques intéressantes.

La demanderesse a découvert de façon surprenante que les dérivés amino-alcools à fonction urée selon l'invention, utilisés dans des compositions capillaires, confèrent aux cheveux un toucher particulièrement doux et facilitent leur démêlage. Ils peuvent être utilisés comme agents de conditionnement des cheveux.

L'objet de l'invention concerne l'utilisation de ces dérivés amino-alcools à fonction urée, comme agents de conditionnement des cheveux dans et pour la préparation de compositions capillaires.

Les dérivés amino-alcools à fonction urée conformes à l'invention répondent à la formule générale (I) suivante: 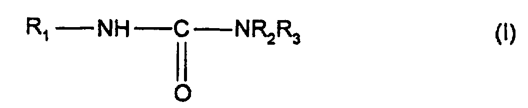 dans laquelle :
- R₁ représente un radical alcoyle, linéaire ou ramifié, saturé ou insaturé ayant de 8 à 22 atomes carbone ;
- R₂ représente un atome d'hydrogène ou un radical alcoyle saturé, linéaire ou ramifié ayant de 1 à 6 atomes de carbone ;
- R₃ représente un groupement alcoyle saturé, monohydroxylé ou polyhydroxylé, linéaire ou ramifié ayant de 1 à 10 atomes carbone.

De préférence, R₁ représente un radical alcoyle, saturé ou insaturé, linéaire ou ramifié, en C₁₀-C₁₈.

De préférence, R₂ représente un hydrogène ou le groupe méthyle.

De préférence, R₃ représente un radical :

―(CH₂)ₙ―(CHOH)ₘ―Z

dans lequel n représente un nombre entier égal à 0 ou 1, m représente un nombre entier compris entre 0 et 5, et Z est un radical alcoyle monohydroxylé ou polyhydroxylé ayant de 1 à 4 atomes de carbone. En particulier, Z est choisi parmi le groupe comprenant les radicaux suivants :

―CH₂OH ―CH₂-CH₂OH ―CH(CH₂OH)₂

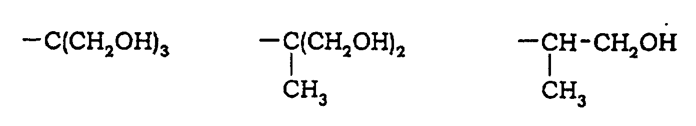

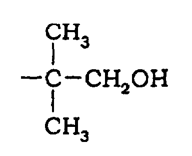

Les radicaux R₃ plus préférentiels sont choisis dans le groupe constitué par :

-CH₂-CHOH-CH₂OH -CH₂-(CHOH)₄CH₂OH

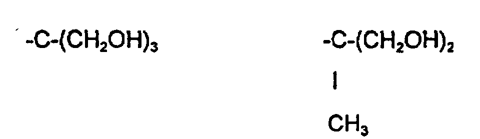

Parmi les composés préférés correspondant à la formule générale (I), on peut notamment citer :
- la N-dodécylaminocarbonyl-N-méthyl-D-glucamine ;
- la N-octylaminocarbonyl-N-méthyl-D-glucamine ;
- la 1-dodécyl-3-(2-hydroxy-1,1-bis-hydroxyméthyl-éthyl)urée ;
- la N-dodécylaminocarbonyl-D-glucamine ;
- la 1-(2,3-dihydroxypropyl)-3-dodécyl-urée ;
- la 1-dodécyl-3-(2-hydroxy-1-hydroxyméthyl-1-méthyl-éthyl)-urée ;
- la 1-(2-éthylhexyl)-3-(2-hydroxy-1,1-bis-hydroxyméthyl-éthyl)urée ;
- la N-(4Z)-octadéc-9-ènyl-aminocarbonyl-N-méthyl-D-glucamine.

Ces composés peuvent être obtenus par réaction, dans un solvant de préférence polaire, d'un aminoalcool de formule R₂-NH-R₃ dans laquelle R₂ et R₃ ont les mêmes significations que celles indiquées précédemment avec un composé imidazolo alkyl carboxamide de formule (II) :

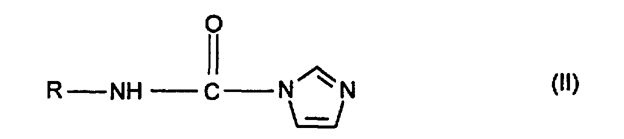

La réaction est généralement effectuée en atmosphère inerte en présence d'un solvant tel que le méthanol, à la température correspondant au reflux du solvant, de préférence entre 40 et 50°C.

Les composés imidazolo alkyl carboxamide de formule (II) sont obtenus selon des méthodes de synthèse connues, notamment dans la référence suivante : Angewandte Chemie, International Edition, Vol 1, n°7, 1962.

Les compositions selon l'invention comprend de préférence 0,001 à 15% en poids de composé de formule (I) par rapport au poids total de la composition.

Le véhicule cosmétiquement acceptable utilisé dans les compositions de l'invention est choisi parmi l'eau; les solvants organiques compatibles avec une application cutanée ou capillaire tels que l'acétone, l'isopropanol, l'éthanol; les triglycérides d'acides gras à 6-24 atomes de carbone, les éthers de glycol, les esters de polyalkylèneglycols et les silicones volatiles ou leurs mélanges.

Les compositions peuvent se présenter sous forme de lotion aqueuse ou hydroalcoolique, monophasique ou polyphasique, de gel monophasique ou polyphasique, d'émulsion, de crème, de dispersion vésiculaire de lipides ioniques ou non ioniques, lesdites vésicules pouvant alors servir en tant qu'agent d'encapsulation pour des ingrédients actifs lipophiles ou hydrophiles, de mousse, de spray.

Les compositions capillaires peuvent se présenter sous forme de shampooing, d'après-shampooing à rincer ou non, de compositions pour permanente, défrisage, coloration ou décoloration, ou encore sous forme de compositions à rincer, à appliquer avant ou après une coloration, une permanente ou un défrisage ou encore entre les deux étapes d'une permanente ou d'un défrisage.

Les compositions cosmétiques ou dermatologiques peuvent par ailleurs contenir des additifs cosmétiques conventionnels choisis parmi les corps gras, les solvants organiques, les silicones, les épaississants, les adoucissants, les tensio-actifs, les polymères anioniques, cationiques, non-ioniques ou amphotères, les agents anti-mousses, les agents conditionneurs du cheveu tels que des protéines, des vitamines, les agents traitants (agents anti-chute, antipelliculaires), les colorants, les parfums, les conservateurs, les agents propulseurs.

Plus précisément, comme corps gras, on peut utiliser une huile ou une cire ou leur mélange, des acides gras, des alcools gras, des esters d'acides gras tels que les triglycérides d'acide gras en C₆ à C₁₈, de la vaseline, de la paraffine, de la lanoline, de la lanoline hydrogénée ou acétylée.

Parmi les huiles, on peut citer les huiles minérales, animales, végétales ou les huiles de synthèse, et notamment l'huile de vaseline, de paraffine, de ricin, de jojoba, de sésame, ainsi que les huiles et les gommes de silicone et les isoparaffines.

Parmi les cires, on peut citer les cires animales, végétales, minérales ou de synthèse, et notamment les cires d'abeilles, de Candelila, les ozokérites, les cires microcristallines ainsi que les cires et résines de silicone.

Parmi les solvants organiques usuellement utilisés dans les compdsitions cosmétiques, on peut citer plus précisément les mono-alcools ou polyalcools inférieurs en C₁ à C₆ comme l'éthanol, l'isopropanol, l'éthylèneglycol, le diéthylènegycol, le propylèneglycol, le glycérol.

Les agents épaississants peuvent être choisis notamment parmi l'alginate de sodium, la gomme arabique, les dérivés cellulosiques tels que la méthylcellulose, l'hydroxy méthylcellulose, l'hydroxyéthylcellulose, l'hydroxypropylcellulose, l'hydroxypropylméthyl cellulose, la gomme de guar ou ses dérivés, la gomme de xanthane, les scléroglucanes, les acides polyacryliques réticulés.

Comme agents tensio-actifs et comme polymères , on peut utiliser tous ceux bien connus de l'état de la technique notamment pour leur utilisation dans des compositions capillaires.

Les compositions peuvent se présenter sous forme de dispersion vésiculaire de lipides amphiphiles ioniques ou non-ioniques. Elles sont préparées notamment en faisant gonfler les lipides dans une solution aqueuse pour former des sphérules dispersées dans le milieu aqueux comme décrit dans STANDISH & WATKINS, J. Mol. Biol., 13,238 (1965) ou dans les brevets FR-A-2.315.991 et FR-A-2.416.008 de la demanderesse. Les différents types de procédés de préparation sont décrits dans "Les liposomes en biologie cellulaire et pharmacologie", Edition INSERM/John Libery Eurotext, 1987, pages 6 à 18.

Le pH des compositions selon l'invention est généralement compris entre 4 et 8 et de préférence entre 5 et 7.

On va maintenant donner à titre d'illustration et sans aucun caractère limitatif plusieurs exemples de préparation de composés selon l'invention ainsi que des exemples de compositions cosmétiques les contenant.

### Exemple 1 Synthèse de la N-dodécylaminocarbonyl-N-méthyl-D-glucamine

### Première étape : Synthèse du 1-N-dodécylaminocarbonyl-imidazole de formule (II) (R₁ = C₁₂H₂₅)

Dans un réacteur, on dissout 49 g de N,N'-carbonyldiimidazole 700 cm³ de diméthylformamide anhydre et on maintient sous atmosphère inerte. On ajoute, de manière à maintenir la température à une valeur inférieure à 30°C, 55g de laurylamine. On maintient sous agitation à température ambiante pendant 8 heures. On verse le mélange réactionnel résultant sur 2 litres d'eau et on filtre le précipité obtenu. Après séchage, on obtient 75 g (rendement 90 %) de 1-N-dodécylaminocarbonyl-imidazole sous forme de poudre blanche.

### Deuxième étape :

### Synthèse de la N-dodécylaminocarbonyl-N-méthyl-D-glucamine de formule (I) (R₁ = C₁₂ H₂₅ , R₂ = CH₃, , R₃ = -CH₂-(CHOH)₄-CH₂OH)

On met en suspension, sous atmosphère inerte, 42g de 1-N-dodécylaminocarbonyl-imidazole et 32 g de N-méthyl-D-glucamine dans 400 cm³ de méthanol. On maintient le mélange réactionnel au reflux sous agitation pendant 4 heures. On refroidit par un bain de glace puis on filtre le précipité. On le lave par 500 cm³ d'eau puis on le sèche. On obtient 49g (rendement 80 %) de N-dodécylaminocarbonyl-N-méthyl-D-glucamine sous forme de poudre blanche.

| **Analyse élémentaire :** | | | | |
|---|---|---|---|---|
| | **C** | **H** | **N** | **O** |
| Calculée (%) | 59,09 | 10,41 | 6,89 | 23,61 |
| Trouvée (%) | 58,83 | 10,45 | 6,96 | 23,37 |

Spectre RMN 1H 200 MHz conforme
Point de fusion : 116,8°C (FP 89)

### Exemple 2 : Synthèse de la 1-dodécyl-3-(2-hydroxy-1,1-bis-hydroxy méthyléthyl)urée de formule (I) R₁ = C₁₂ H₂₅, R₂ = H, R₃ =-C-(CH₂OH)₃)

On procède selon le même procédé décrit dans l'exemple 1 à partir de 5,2 g de trihydroxyméthylaminométhane de formule R₂-NH-R₃ correspondant où R₂ désigne H et R₃ désigne -C-(CH₂OH)₃ et 8 g de 1-N-dodécylaminocarbonyl-imidazole de formule (II) correspondant où R₁ désigne C₁₂ H₂₅ dans 100 cm³ de méthanol.

| **Analyse élémentaire :** | | | | |
|---|---|---|---|---|
| | **C** | **H** | **N** | **O** |
| Calculée (%) | 61,41 | 10,91 | 8,43 | 19,25 |
| Trouvée (%) | 61,40 | 11,08 | 8,29 | 19,12 |

Spectre RMN 1H 200 MHz conforme
Point de fusion : 132,4°C (FP 89)

### Exemple 3 : Synthèse de la N-dodécylaminocarbonyl-D-glucamine de formule (I) (R₁ = C₁₂ H₂₅ , R₂ = H, R₃ = -CH₂-(CHOH)₄-CH₂OH)

On procède selon le même procédé décrit dans l'exemple 1 à partir de 6,1 g de D-glucamine de formule R₂-NH-R₃ correspondant où R₂ désigne H et R₃ désigne -CH₂-(CHOH)₄-CH₂OH) et 7g de 1-N-dodécylaminocarbonyl-imidazole de formule (II) correspondant où R₁ désigne C₁₂ H₂₅ dans 100 cm³ de méthanol.

| **Analyse élémentaire :** | | | | |
|---|---|---|---|---|
| | **C** | **H** | **N** | **O** |
| Calculée (%) | 58,14 | 10,27 | 7,14 | 24,46 |
| Trouvée (%) | 58,09 | 10,21 | 7,18 | 24,73 |

Spectre RMN 1H 200 MHz conforme
Point de fusion : 161,5°C (FP 89)

### Exemple 4 : Synthèse de la 1-(2,3-dihydroxypropyl)-3-dodécyl-urée de formule (I) (R₁ = C₁₂ H₂₅ , R₂ = H, R₃ = -CH₂ -CHOH-CH₂OH)

On procède selon le même procédé décrit dans l'exemple 1 à partir 2,3 g de 3-amino-1,2-propanediol de formule R₂-NH-R₃ correspondant où R₂ désigne H et R₃ désigne -CH₂-CHOH-CH₂OH et de 7 g de1-N-dodécyaminocarbonyl-imidazole de formule (II) correspondant où R₁ désigne C₁₂H₂₅ dans 100 cm³ de méthanol.

| **Analyse élémentaire :** | | | | |
|---|---|---|---|---|
| | **C** | **H** | **N** | **O** |
| Calculée (%) | 63,54 | 11,33 | 9,26 | 15,87 |
| Trouvée (%) | 63,51 | 11,45 | 9,14 | 16,09 |

Spectre RMN 1H 200 MHz conforme
Point de fusion : 121,5°C (FP 89)

### Exemple 5 : Synthèse de la 1-dodécyl-(2-hydroxy-1-hydroxyméthyl-1-méthyl-éthyl)-urée de formule (I)

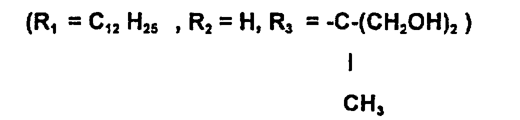

On procède selon le même procédé décrit dans l'exemple 1 à partir de 3,5 g de 2-amino-2-méthyl-1,3-propanediol de formule R₂-NH-R₃ correspondant où R₂ désigne H et R₃ désigne : 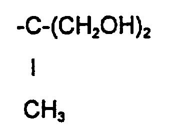 et de 7 g de1-N-dodécylaminocarbonyl-imidazole de formule (II) correspondant où R₁ désigne C₁₂ H₂₅ dans 100 cm³ de méthanol.

| **Analyse élémentaire :** | | | | |
|---|---|---|---|---|
| | **C** | **H** | **N** | **O** |
| Calculée (%) | 64,52 | 11,47 | 8,85 | 15,17 |
| Trouvée (%) | 64,52 | 11,43 | 8,84 | 15,00 |

Spectre RMN 1H 200 MHz conforme
Point de fusion : 60,1°C (FP 89)

### Exemple 6 : Synthèse de la 1-(2-éthylhexyl)-3-(2-hydroxy-1,1-bis-hydroxyméthyl éthyl)urée de formule (I) (R₁ = C₄ H₇ -CH-(C₂ H₅)-CH₂), R₂ = H, R₃ = -C-(CH₂OH)₃)

On procède selon le même procédé décrit dans l'exemple 1 à partir de 14 g de trihydroxy méthylaminométhane de formule R₂-NH-R₃ correspondant où R₂ désigne H et R₃ désigne -C-(CH₂OH)₃ et de 25 g de1-N-2-éthyl-hexylaminocarbonyl-imidazole de formule (II) correspondant où R₁ désigne C₄ H₇-CH-(C₂ H₅)-CH₂ dans 200 cm³ de méthanol.

| **Analyse élémentaire :** | | | | |
|---|---|---|---|---|
| | **C** | **H** | **N** | **O** |
| Calculée (%) | 56,50 | 10,21 | 10,14 | 23,16 |
| Trouvée (%) | 56,56 | 10,35 | 10,23 | 23,38 |

Spectre RMN 1H 200 Mhz conforme
Point de fusion : 155,8°C (FP 89)

### Exemple 7 : Synthèse de la N-(4Z)-octadéc-9-ènyl-aminocarbonyl-N-méthyl-D-glucamine. de formule (I) (R₁ = C₈ H₁₇-CH=CH-C₈H₁₆, R₂ = CH₃,, R₃ = -CH₂-(CHOH)₄ -CH₂OH)

On procède selon le même procédé décrit dans l'exemple 1 à partir de N-méthyl-D-glucamine de formule R₂-NH-R₃ correspondant où R₂ désigne CH₃ et R₃ désigne -CH₂-(CHOH)₄ CH₂OH et 48 g 1-N-octadéc-9-ènyl-aminocarbonyl-imidazole de formule (II) correspondant où R₁ désigne C₈ H₁₇-CH=CH-C₈ H₁₆ dans 300 cm³ de méthanol.

| **Analyse élémentaire :** | | | | |
|---|---|---|---|---|
| | **C** | **H** | **N** | **O** |
| Calculée (%) | 63,90 | 10,72 | 5,73 | 19,64 |
| Trouvée (%) | 64,07 | 10,75 | 5,85 | 19,90 |

Spectre RMN 1H 200 MHz conforme
Point de fusion 116,2°C (FP 89)

### Exemple 8 : Synthèse de la N-octylaminocarbonyl-N-méthyl-D-glucamine de formule (I) (R₁ = C₈ H₁₇, R₂ = CH₃, , R₃ = -CH₂-(CHOH)₄ -CH₂OH)

### 1 Synthèse du -N-octylaminocarbonyl-imidazole

On dissout 80 g de N,N'-carbonyldiimidazole dans 900 cm3 de diméthylformamide anhydre et on maintient sous atmosphère inerte. On ajoute 63 g d'octyl en maintenant la température en dessous de 30°C. On maintient sous agitation à température ambiante pendant 4 heures. On verse sur 3 litres pour extraire par 4 fois 400 cm3 d'acétate d'éthyle. On sèche, on filtre et on évapore jusqu'à obtenir 84 g de produit sous forme d'une huile pâteuse (Rendement : 86%). Le Spectre RMN 1H 200 MHz est conforme.

### 2 Synthèse de la N-octylaminocarbonyl-N-méthyl-D-glucamine

On met en suspension, sous atmosphère inerte, 47g de 1-N-octylaminocarbonyl-imidazole et 42 g de N-méthyl-D-glucamine dans 300 cm³ de méthanol. On maintient le mélange réactionnel au reflux sous agitation pendant 3 heures. On refroidit par un bain de glace puis on filtre le précipité. On le recristallise dans 300 cm³ d'éthanol. On obtient 40g (rendement 55 %) de N-octylaminocarbonyl-N-méthyl-D-glucamine sous forme de poudre blanche.
Spectre RMN 1H 200 MHz conforme
Spectre de masse conforme
Point de fusion : 111,4°C (FP 89)

| **Analyse élémentaire :** | | | | |
|---|---|---|---|---|
| | **C** | **H** | **N** | **O** |
| Calculée (%) | 54,84 | 9,78 | 7,99 | 27,39 |
| Trouvée (%) | 53,58 | 9,71 | 8,25 | 28,34 |

### Exemple 9 : Exemple comparatif

### Mesure de la perte insensible en eau (PIE)

Cette mesure s'effectue à l'aide d'un évaporimètre (Servomed) qui détermine quantitativement l'évaporation d'eau, c'est-à-dire un transport d'eau par diffusion, à partir d'un échantillon de Stratum Corneum obturant une capsule cylindrique contenant de l'eau, le tout étant placé dans une chambre à température et humidité relatives contrôlées.

Des capteurs permettent de mesurer la pression partielle de vapeur d'eau en deux points situés à des distances différentes de l'échantillon.

On détermine ainsi le gradient de pression partielle de vapeur d'eau entre les deux points, et donc le taux d'évaporation conformément à la loi de Fick.

Cette mesure est effectuée pour :
a) la N-dodécylaminocarbonyl-N-méthyl-D-glucamine (composé de l'exemple 1) ;
b) la N-dodécanoyl-N-méthyl-D-glucamine, homologue amide du composé de l'exemple 1, connu pour être un agent hydratant que l'on nommera reférence A ;
c) la N-dodécyloxycarbonyl-N-méthyl-D-glucamine, homologue carbamate du composé de l'exemple 1, connu pour être un agent hydratant que l'on nommera référence B.

Les composés sont préparés à raison de 1% dans un mélange de dichlorométhane/méthanol (2/1 v/v), puis appliqués sur des morceaux de Stratum Comeum délipidé.

On mesure, 20 heures après l'application, la perte insensible en eau (PIE) pour chaque composé.

On obtient les résultats suivants

| Composé | exemple 1 | référence A | référence B |
|---|---|---|---|
| P.I.E. | - 21 +/-2 | - 11 +/-3 | -9 +/-2 |

On constate donc que l'application du composé selon l'invention permet de réduire de façon plus importante l'évaporation de l'eau contenue dans le Stratum Corneum par rapport à ses homologues amide et carbamate.

### Exemple 10 : Crème après-shampooing

- N-dodécylaminocarbonyl-N-méthyl-D-glucamine 2 g MA
- Chlorure de distéaryldiméthylammonium 2 g
- Hydroxyéthylcellulose vendu sous le nom de NATROSOL 250 HHR par AQUALON 1 g
- Conservateur, parfum, colorant qs
- Eau qsp 100 g
pH ajusté à 6 par NaOH

On obtient une crème fluide laiteuse qui après application confère aux cheveux un toucher doux et un démêlage facile.

## Revendications

1. Utilisation d'au moins un dérivé aminoalcool à fonction urée de formule (I) : 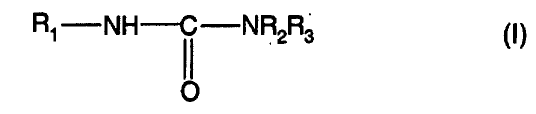 dans laquelle:
- R₁ représente un radical alcoyle, linéaire ou ramifié, saturé ou insaturé ayant de 8 à 22 atomes carbone ;
- R₂ représente un atome d'hydrogène ou un radical alcoyle saturé, linéaire ou ramifié ayant de 1 à 6 atomes de carbone ;
- R₃ représente un groupement alcoyle saturé, monohydroxylé ou polyhydroxylé, linéaire ou ramifié ayant de 1 à 10 atomes carbone,
comme agent de conditionnement des cheveux dans une composition cosmétique capillaire.

2. Utilisation selon la revendication 1, dans laquelle ledit dérivé de formule (I) est présent en une quantité de 0,001 à 15% en poids par rapport au poids total de la composition.

3. Utilisation selon l'une des revendications précédentes, dans laquelle, dans la formule (I), R₁ représente un radical alcoyle, linéaire ou ramifié, saturé ou insaturé en C₁₀-C₁₈.

4. Utilisation selon l'une des revendications précédentes, dans laquelle, dans la formule (I), R₂ représente un hydrogène ou le groupe méthyle.

5. Utilisation selon l'une des revendications précédentes, dans laquelle, dans la formule (I), R₃ représente un radical:
―(CH₂)ₙ-(CHOH)ₘ―Z
dans lequel n représente un nombre entier égal à 0 ou 1, m représente un nombre entier compris entre 0 et 5, et Z est un radical alcoyle monohydroxylé ou polyhydroxylé ayant de 1 à 4 atomes de carbone.

6. Utilisation selon la revendication 5, dans laquelle Z est choisi parmi les radicaux suivants:
―CH₂OH ―CH₂-CH₂OH ―CH(CH₂OH)₂

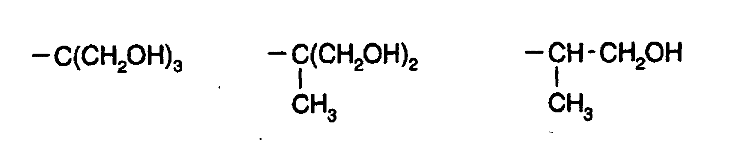

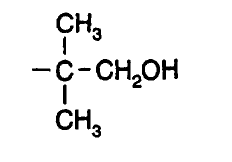

7. Utilisation selon l'une des revendications précédentes, dans laquelle R₃ est choisi dans le groupe constitué par:
-CH₂-CHOH-CH₂OH, -CH₂-(CHOH)₄CH₂OH, -C-(CH₂OH)₃
et

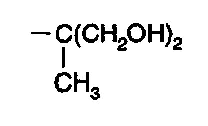

8. Utilisation selon l'une des revendications précédentes, dans laquelle les composés de formule. (I) sont choisis parmi:
- la N-dodécylaminocarbonyl-N-méthyl-D-glucamine ;
- la N-octylaminocarbonyl-N-méthylglucamine ;
- la 1-dodécyl-3-(2-hydroxy-1,1-bis-hydroxyméthyl-éthyl)urée ;
- la N-dodécylaminocarbonyl-D-glucamine ;
- la 1-(2,3-dihydroxypropyl)-3-dodécyl-urée ;
- la 1-dodécyl-3-(2-hydroxy-1-hydroxyméthyl-1-méthyl-éthyl)-urée ;
- la 1-(2-éthylhexyl)-3-(2-hydroxy-1,1-bis-hydroxyméthyl-éthyl)urée ;
- la N-(4Z)-octadéc-9-ènyl-aminocarbonyl-N-méthyl-D-glucamine.

9. Utilisation selon l'une des revendications précédentes, dans laquelle la composition capillaire se présente sous forme de shampooing, d'après-shampooing à rincer ou non ; de composition pour permanente, défrisage, coloration ou décoloration ; de compositions à rincer, à appliquer avant ou après une coloration, une permanente ou un défrisage ou encore entre les deux étapes d'une permanente ou d'un défrisage.

## Patentansprüche

1. Verwendung mindestens eines Aminoalkoholderivats mit Harnstoffgruppe der Formel (I): 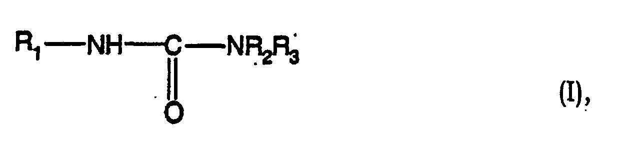 worin bedeuten:
- R₁ eine geradkettige oder verzweigte, gesättigte oder ungesättigte Alkylgruppe mit 8 bis 22 Kohlenstoffatomen;
- R₂ Wasserstoff oder eine gesättigte, geradkettige oder verzweigte Alkylgruppe mit 1 bis 6 Kohlenstoffatomen;
- R₃ eine gesättigte, ein- oder mehrfach hydroxylierte, geradkettige oder verzweigte Alkylgruppe mit 1 bis 10 Kohlenstoffatomen,
als Konditioniermittel für das Haar in kosmetischen Zusammensetzungen für das Haar.

2. Verwendung nach Anspruch 1, **dadurch gekennzeichnet, daß** das Derivat der Formel (I) in einer Menge von 0,001 bis 15 Gew.-%, bezogen auf das Gesamtgewicht der Zusammensetzung, vorliegt.

3. Verwendung nach einem der vorhergehenden Ansprüche, wobei in der Formel (I) R₁ eine geradkettige oder verzweigte, gesättigte oder ungesättigte C₁₀₋₁₈-Alkylgruppe bedeutet.

4. Verwendung nach einem der vorhergehenden Ansprüche, wobei in der Formel (I) R₂ Wasserstoff oder Methyl bedeutet.

5. Verwendung nach einem der vorhergehenden Ansprüche, wobei die Gruppe R₃ in der Formel (I) die folgende Gruppe bedeutet:
―(CH₂)ₙ―(CHOH)ₘ―Z,
wobei n 0 oder 1 bedeutet, m 0 oder eine ganze Zahl im Bereich von 0 bis 5 ist und Z eine ein- oder mehrfach hydroxylierte Alkylgruppe mit 1 bis 4 Kohlenstoffatomen bedeutet.

6. Verwendung nach Anspruch 5, wobei Z unter den folgenden Gruppen ausgewählt ist:
―CH₂OH ―CH₂-CH₂OH ―CH(CH₂OH)₂

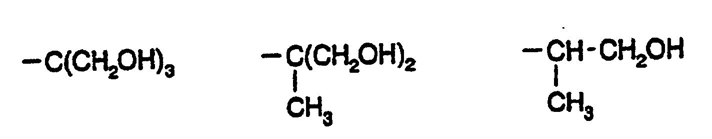

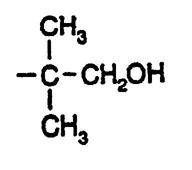

7. Verwendung nach einem der vorhergehenden Ansprüche, wobei R₃ unter den folgenden Verbindungen ausgewählt ist:
-CH₂-CHOH-CH₂OH, -CH₂-(CHOH)₄CH₂OH, -C-(CH₂OH)₃
und

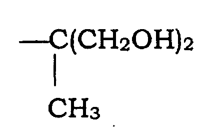

8. Verwendung nach einem der vorhergehenden Ansprüche, wobei die Verbindungen der Formel (I) ausgewählt sind unter:
- N-Dodecylaminocarbonyl-N-methyl-D-glucamin;
- N-Octylaminocarbonyl-N-methylglucamin;
- 1 -Dodecyl-3-(2-hydroxy-1,1-bis-hydroxymethylethyl)-harnstoff;
- N-Dodecylaminocarbonyl-D-glucamin;
- 1,(2,3-Dihydroxypropyl)-3-dodecyl-harnstoff;
- 1-Dodecyl-3-(2-hydroxy-1-hydroxymethyl-1-methylethyl)-harnstoff;
- 1-(2-Ethylhexyl)-3-(2-hydroxy-1,1-bis-hydroxymethyl-ethyl)-harnstoff; und
- N-(4Z)-Octadec-9-enyl-aminocarbonyl-N-methyl-D-glucamin.

9. Verwendung nach einem der vorhergehenden Ansprüche, wobei die Zusammensetzung für das Haar als Haarwaschmittel, Haarpflegemittel, das ausgespült wird oder im Haar verbleibt, Zusammensetzung für permanente Verformungen, Entkräuselungen, Färbungen oder Entfärbungen; und Zusammensetzungen, die ausgespült werden und vor oder nach einer Färbung, permanenten Verformung oder Entkräuselung oder zwischen den beiden Schritten einer dauerhaften Verformung oder Entkräuselung aufgetragen werden.

## Claims

1. Use of at least one amino alcohol derivative containing a urea function of formula (I) : 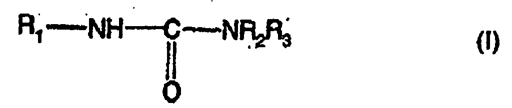 in which:
- R₁ represents a linear or branched, saturated or unsaturated alkyl radical containing from 8 to 22 carbon atoms;
- R₂ represents a hydrogen atom or a saturated, linear or branched alkyl radical containing from 1 to 6 carbon atoms;
- R₃ represents a saturated, monohydroxylated or polyhydroxylated, linear or branched alkyl group containing from 1 to 10 carbon atoms,
as a hair conditioner in a cosmetic hair composition.

2. Use according to Claim 1, in which the said derivative of formula (I) is present in an amount of from 0.001% to 15% by weight relative to the total weight of the composition.

3. Use according to either of the preceding claims, in which, in formula (I), R₁ represents a linear or branched, saturated or unsaturated C₁₀-C₁₈ alkyl radical.

4. Use according to one of the preceding claims, in which, in formula (I), R₂ represents a hydrogen or a methyl group.

5. Use according to one of the preceding claims, in which, in formula (I), R₃ represents a radical:
- (CH₂)ₙ-(CHOH)ₘ-Z
in which n represents an integer equal to 0 or 1, m represents an integer between 0 and 5, and Z is a monohydroxylated or polyhydroxylated alkyl radical containing from 1 to 4 carbon atoms.

6. Use according to Claim 5, in which Z is chosen from the following radicals:
-CH₂OH -CH₂-CH₂OH -CH(CH₂OH)₂

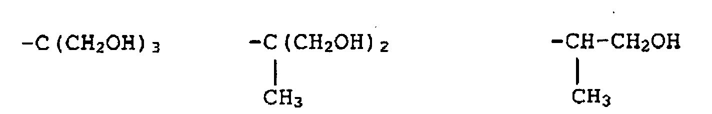

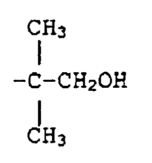

7. Use according to one of the preceding claims, in which R₃ is chosen from the group consisting of:
-CH₂-CHOH-CH₂OH, -CH₂- (CHOH)₄CH₂OH, -C- (CH₂OH)₃
and

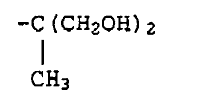

8. Use according to one of the preceding claims, in which the compounds of formula (I) are chosen from:
- N-dodecylaminocarbonyl-N-methyl-D-glucamine;
- N-octylaminocarbonyl-N-methylglucamine;
- 1-dodecyl-3-[2-hydroxy-1,1-bis(hydroxymethyl)ethyl]-urea;
- N-dodecylaminocarbonyl-D-glucamine;
- 1-(2,3-dihydroxypropyl)-3-dodecylurea;
- 1-dodecyl-3-(2-hydroxy-1-hydroxymethyl-1-methylethyl)urea;
- 1-(2-ethylhexyl)-3-[2-hydroxy-1,1-bis(hydroxymethyl)ethyl]urea;
- N-(4Z)-octadec-9-enylaminocarbonyl-N-methyl-D-glucamine.

9. Use according to one of the preceding claims, in which the hair composition is in the form of a shampoo or a rinse-out or leave-in conditioner; a permanent-waving, hair-straightening, dyeing or bleaching composition; a rinse-out composition to be applied before or after dyeing, permanent-waving or straightening the hair, or alternatively between the two steps of a permanent-waving or hair-straightening operation.
